# EUROPÄISCHE PATENTSCHRIFT

(11) **EP 1 208 094 B1**
(45) Veröffentlichungstag und Bekanntmachung des Hinweises auf die Patenterteilung: **03.11.2004**
(21) Anmeldenummer: 00964033.5
(22) Anmeldetag: 23.08.2000
(51) Int. Cl.: C07D 307/33

(54) **VERFAHREN ZUR HERSTELLUNG VON GAMMA-BUTYROLACTON**
METHOD FOR PRODUCING GAMMA-BUTYROLACTONE
PROCEDE DE PREPARATION DE GAMMA-BUTYROLACTONE

(30) Priorität: 01.09.1999 DE 19941569
(43) Veröffentlichungstag der Anmeldung: 29.05.2002
(73) Patentinhaber: BASF AKTIENGESELLSCHAFT, 67056 Ludwigshafen (DE)
(72) Erfinder: FISCHER, Rolf, 69121 Heidelberg (DE); PINKOS, Rolf, 67098 Bad Dürkheim (DE)
(86) Internationale Anmeldenummer: PCT/EP2000/008216
(87) Internationale Veröffentlichungsnummer: WO 2001/016126

(56) Entgegenhaltungen:
- EP-A- 0 584 408
- EP-A- 0 628 552
- EP-A- 0 848 991
- WO-A-92/00973

## Beschreibung

Die Erfindung betrifft ein Verfahren zur Herstellung von gamma-Butyrolacton (GBL) durch Umsetzung einer 1,4-Butandiol enthaltenden Reaktionsmischung an einem Kupfer-Katalysator.

Verfahren zur Herstellung von GBL aus 1,4-Butandiol sind seit langem bekannt. In K. Weissermel, H.-J. Arpe, Industrielle Organische Chemie, VCH Verlagsgesellschaft, D 69451 Weinheim, 1994, Seite 112 ist die Dehydrocyclisierung von 1,4-Butandiol an Kupfer-Katalysatoren bei Temperaturen von 200 bis 250°C beschrieben.

EP-A 628 552 offenbart ein Verfahren zur katalytischen Dehydrierung von Diolen mit 3 bis 10 Kohlenstoffatomen an einem Kupfer-Chrom-Katalysator in einem zweistufigen Verfahren. In den Beispielen 1 und 2 wird 1,4-Butandiol zu γ-Butyrolacton dehydriert.

Auch PCT/WO 92/000973 offenbart ein Verfahren zur Herstellung von Lactonen wie γ-Butyrolacton aus Diolen an Kupfer/Chrom-Katalysatoren. Die Katalysatoren sollen eine bestimmte Partikelgröße aufweisen.

Ein weiteres Verfahren zur katalytischen Dehydrierung von 1,4-Butandiol zu γ-Butyrolacton an Kupfer/Chrom-Katalysatoren ist aus EP-A 584 408 bekannt. Der nach Abtrennung von γ-Butyrolacton vom Reaktionsansatz verbleibende Rest der Reaktionsmischung wird zurückgeführt.

Nachteilig an diesem Verfahren zur Herstellung von GBL ist, daß das eingesetzte 1,4-Butandiol vor dem Einsatz zunächst gereinigt werden muß. Die Reinigung erfolgt meist durch aufwendige mehrstufige Destillation, wobei unerwünschte leicht- und/oder schwersiedende Bestandteile, einschließlich Wasser, abgetrennt werden. Anschließend wird dieses wasserfreie Rein-Butandiol zu GBL cyclisiert, wobei unerwünschte Nebenprodukte entstehen. Daher muß das erkaltete GBL nach der Umsetzung erneut destillativ gereinigt werden. Es müssen also zwei vergleichbare, aufwendige Reinigungs- und Abtrennungsschritte durchgeführt werden.

Die Aufgabe der vorliegenden Erfindung ist es daher, ein Verfahren zur Herstellung von GBL aus 1,4-Butandiol bereitzustellen, bei dem das eingesetzte Butandiol nicht vorgereinigt werden muß und die Bildung unerwünschter Nebenprodukte vermieden wird.

Diese Aufgabe wird erfindungsgemäß gelöst durch ein Verfahren zur Herstellung von GBL durch Umsetzung von 1,4-Butandiol an einem Kupfer-Katalysator, dadurch gekennzeichnet, daß eine 1,4-Butandiol enthaltende Reaktionsmischung, die weitere von 1,4-Butandiol verschiedene Alkohole und Wasser enthält, eingesetzt wird.

Das erfindungsgemäße Verfahren hat den Vorteil, daß die eingesetzten 1,4-Butandiol enthaltenden Reaktionsmischungen nicht vor der Umsetzung zu GBL vorgereinigt werden müssen. 1,4-Butandiol kann in Gegenwart von weiteren Alkoholen zu GBL cyclisiert werden, ohne daß nennenswerte Mengen an Nebenprodukten entstehen. Damit entfällt die aufwendige Vorreinigung, so daß Kosten gespart werden können.

Die bei der Umsetzung als Zulauf eingesetzten 1,4-Butandiol enthaltenden Reaktionsmischungen können durch bekannte Verfahren erhalten werden.

So kann beispielsweise eine 1,4-Butandiol enthaltende Reaktionsmischung eingesetzt werden, die nach dem Reppe-Verfahren aus Acetylen und Formaldehyd und anschließende Hydrierung des entstandenen 1,4-Butindiols gewonnen wird, oder durch Acetoxylierung bzw. Chlorierung von Butadien durch Hydrierung von 4-Hydroxybutyraldehyd und 2,3- oder 2,5- Dihydrofuran.

Bevorzugt wird der Hydrieraustrag der Hydrierung einer Verbindung ausgewählt aus 1,4-Butindiol, 4-Hydroxybutyraldehyd, 2,3- oder 2,5-Dihydrofuran, Maleinsäure, Maleinsäuremonoestern, Maleinsäurediestern, Maleinsäureanhydrid und/oder einem bei deren Hydrierung entstehenden Zwischenprodukt als Zulauf eingesetzt. Solche Zwischenprodukte sind beispielsweise Bernsteinsäureanhydrid, Bernsteinsäure oder Bernsteinsäurediester. Besonders bevorzugt wird der Hydrieraustrag der Hydrierung von 1,4-Butindiol, 4-Hydroxybutyraldehyd, Maleinsäure oder Maleinsäurediestern als Zulauf bei der Umsetzung zu GBL eingesetzt.

Die Hydrierung kann in bekannter Weise in der Gas- oder Flüssigphase durchgeführt werden. Beispielsweise kann Maleinsäuredimethylester durch Hydrierung an einem Katalysator, z.B. Kupferchromit, unter erhöhtem Druck und erhöhter Temperatur in der Gasphase umgesetzt werden. Der gewonnene Hydrieraustrag, der als Zulauf in der erfindungsgemäßen Umsetzung eingesetzt wird, enthält im allgemeinen 5 - 95 Gew.-% Butandiol und 15 - 95 Gew.-% Alkohol, bevorzugt 10 bis 70 Gew.-% Butandiol und 5 bis 70 Gew.-% Alkohol, besonders bevorzugt 15 bis 60 Gew.-% Butandiol und 15 bis 50 Gew.-% Alkohol. Daneben können Produkte wie Bernsteinsäurediester, Wasser, 4-Hydroxybuttersäure und deren Ester sowie 4-Hydroxybutyraldehyd im Bereich bis zu 30 Gew.-% enthalten sein. Der Gehalt an Bernsteinsäurediester ist im allgemeinen für das Verfahren unkritisch. Weiterhin kann Wasser in einem Gehalt von im allgemeinen unter 5 Gew.-%, bevorzugt unter 2 Gew.-%, besonders bevorzugt unter 1 Gew.-% vorhanden sein, sowie geringe Mengen weiterer Verbindungen. Es ist möglich, daß im Hydrieraustrag bereits GBL vorliegt, wobei der GBL-Gehalt für das Verfahren nicht kritisch ist und z.B. zwischen 10 und 30 Gew.-% liegen kann. Wird der Hydrieraustrag der Hydrierung von 1,4-Butindiol, 4-Hydroxybutyraldehyd oder Maleinsäure als Butandiolquelle eingesetzt, ist neben Wasser als Alkoholkomponente im allgemeinen noch n-Butanol vorhanden. Das Molverhältnis von n-Bu-tanol zu 1,4-Butandiol kann in diesen Hydrierausträgen beispielsweise 0,5 : 100 bis 5 : 100 betragen. Anstelle des gesamten Hydrieraustrages kann auch nur ein Teilstrom des Hydrieraustrages der Umsetzung zu GBL zugeführt werden. Das Reaktionsprodukt der Umsetzung zu GBL kann denselben Aufarbeitungskolonnen zugeführt werden wie der nicht weiter umgesetzte Teilstrom des Hydrieraustrages, da beide ähnliche Verunreinigungen und Nebenprodukte aufweisen. So brauchen nicht für vergleichbare Trennaufgaben unterschiedliche Apparaturen betrieben zu werden.

Die Reaktionsmischung in dem erfindungsgemäßen Verfahren zur Herstellung von GBL enthält im allgemeinen aliphatische Alkohole, die bevorzugt einwertig sind. Insbesondere bevorzugt sind aliphatische Alkohole mit 1 bis 4 Kohlenstoffatomen, wie Methanol, Ethanol, iso- und n-Propanol und n-Butanol, enthalten.

Die Umsetzung der 1,4-Butandiol enthaltenden Reaktionsmischung erfolgt erfindungsgemäß bei 200 bis 350°C, bevorzugt bei 230 bis 330°C, besonders bevorzugt bei 230 bis 240°C. Sie wird in einem Druckbereich von 0,5 bis 10 bar, bevorzugt 0,8 bis 5 bar, besonders bevorzugt bei 1 bis 3 bar durchgeführt.

In Abhängigkeit von den gewählten Temperatur- und Druckbedingungen können das gebildete GBL sowie der Alkohol und Wasser von der flüssigen in die gasförmige Phase übergehen oder in der flüssigen Phase verbleiben. Bei niedrigen Reaktionsdrucken wird der Produktstrom den Reaktor gasförmig verlassen. Der bei der Reaktion freiwerdende Wasserstoff kann als Trägergas für den Edukt-/Produktstrom verwendet werden.

In dem erfindungsgemäßen Verfahren wird ein Katalysator verwendet, der Kupfer alleine oder zusammen mit mindestens einem Metall der VIII. Nebengruppe des Periodensystems, bevorzugt aufgebracht auf einen Träger, umfaßt.

Prinzipiell können alle Metalle der VIII. Nebengruppe des Periodensystems zusätzlich zum Kupfer eingesetzt werden. Vorzugsweise werden jedoch Nickel, Palladium, Kobalt oder Ruthenium oder ein Gemisch aus zwei oder mehreren davon eingesetzt.

Der Kupfergehalt des Katalysators beträgt im allgemeinen 1 bis 80 Gew.-%, vorzugsweise 3 bis 50 Gew.-% und insbesondere bevorzugt 5 bis 40 Gew.-%, jeweils bezogen auf das Gesamtgewicht des Katalysators und berechnet als Metall.

Die bevorzugt erfindungsgemäß verwendeten geträgerten Kupfer-Katalysatoren können technisch durch Auftragen des Kupfers und gegebenenfalls mindestens eines Metalles der VIII. Nebengruppe des Periodensystems auf einen Träger hergestellt werden.

Das Auftragen kann durch Tränken des Trägers in wäßrigen Metallsalzlösungen, wie zum Beispiel wäßrigen Kupferlösungen, durch Aufsprühen entsprechender Metallsalzlösungen auf den Träger, durch Fällung der Metalle und Trägermaterialien oder durch andere geeignete Verfahren erreicht werden.

Als Kupfersalze oder Metallsalze der VIII. Nebengruppe des Periodensystems eignen sich die Nitrate, Nitrosylnitrate, Halogenide, Carbonate, Carboxylate, Acetylacetonate, Nitritokomplexe oder Ammoniumkomplexe der entsprechenden Metalle, wobei die Nitrate, Carboxylate, Ammoniakate bevorzugt sind. Dabei können Kupfersalze und Metallsalze der VIII. Nebengruppe des Periodensystems beim Auftragen durch Tränken gleichzeitig oder nacheinander auf den Träger aufgebracht werden.

Die mit der Metallsalzlösung beschichteten oder getränkten Träger werden anschließend, vorzugsweise bei Temperaturen zwischen 100 und 150°C getrocknet und wahlweise bei Temperaturen zwischen 200°C und 600°C, vorzugsweise bei 350 bis 450°C calciniert. Bei getrennter Auftränkung wird der Katalysator nach jedem Tränkschritt getrocknet und wahlweise calciniert.

Die Herstellung der erfindungsgemäß verwendeten Katalysatoren durch Fällung (Fällungskatalysatoren) erfolgt, in dem man Kupfer und gegebenenfalls die Metalle der VIII. Nebengruppe des Periodensystems aus deren Salzlösungen, z.B. als schwerlösliche Hydroxyde, Oyxdhydrate, barische Salze oder Carbonate ausfällt, die erkalteten Niederschläge anschließend trocknet und diese dann durch Calcinierung bei im allgemeinen 300 bis 700°C, insbesondere bei 400 bis 600°C, in die entsprechenden Oxide, Mischoxide und/oder gemischtvalentigen Oxide umwandelt. Bei der Herstellung der erfindungsgemäßen Fällungskatalysatoren kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden.

Anschließend werden die Fällungskatalysatoren und die beschichteten und getrockneten sowie wahlweise calcinierten Katalysatoren durch Behandlung in einem Wasserstoff enthaltenden Gasstrom bei Temperaturen von 30 bis 600°C, vorzugsweise 150 bis 450°C, aktiviert, wobei diese Aktivierung vorzugsweise in situ zu Beginn der Reaktion oder vor ihrer Verwendung separat erfolgen kann.

Als Trägermaterialien können im allgemeinen die Oxide des Aluminiums und Titans, Siliciumdioxid, Zirkoniumdioxid, Calciumoxid,Bariumoxid, Magnesiumoxid und Chromtrioxid (Cr₂O₃) verwendet werden. Bevorzugte Trägermaterialien sind die Oxide des Aluminiums, des Titans, Siliciumdioxid, Calciumoxid und Magnesiumoxid. Selbstverständlich können auch Mischungen verschiedener Trägermaterialien als Träger dienen.

Die erfindungsgemäße Umsetzung wird bevorzugt an fest angeordneten Katalysatoren in der Gasphase durchgeführt werden. Beispielsweise kann die Umsetzung von 1,4-Butandiol zu GBL in der Gasphase in einem Wirbelbettreaktor oder einem Rohrreaktor erfolgen. Der Rohrreaktor kann in der Riesel- oder Sumpffahrweise betrieben werden. Der bei der Umsetzung freiwerdende Wasserstoff kann als Trägergas für den Edukt/Produktstrom verwendet werden. Wird der Rohrreaktor in Sumpffahrweise betrieben, können sich die in der Reaktionsmischung enthaltenen Hochsieder aus der Hydrierung von Maleinsäure oder Maleinsäurederivaten im Reaktionssumpf ansammeln und als Teilstrom ausgeschleust werden. Dieser kann dann verworfen oder in frühere Verfahrensstufen, zum Beispiel der Hydrierung, zurückgeführt werden.

Die Zulaufmenge beträgt pro Liter Katalysator im allgemeinen 0,05 bis 15 kg/h, bevorzugt 0,1 bis 1 kg/h, besonders bevorzugt 0,2 bis 0,8 kg/h.

Der Umsatz des in der Reaktionsmischung vorhandenen 1,4-Butandiols zu GBL beträgt im allgemeinen 99 bis 100 %. Somit entspricht der Austrag nach der Umsetzung von 1,4-Butandiol zu GBL (Cyclisierungsaustrag) im wesentlichen seiner Zulaufzusammensetzung, mit dem Unterschied, daß das im Zulauf enthaltene 1,4-Butandiol zu GBL und Wasserstoff umgesetzt wurde. Der Cyclisierungsaustrag enthält im allgemeinen GBL, Wasser und Alkohol.

Anhand der Zusammensetzung des Cyclisierungsaustrages wird deutlich, daß bei der Umsetzung von 1,4-Butandiol enthaltenden Reaktionsmischungen zu GBL an Kupfer-Katalysatoren in Gegenwart von wasser und weiteren Alkoholen keine nennenswerte Bildung von Nebenprodukten auftritt.

Der Cyclisierungsaustrag kann mit dem Fachmann bekannten Methoden destillativ aufgearbeitet werden.

GBL ist ein gesuchtes Produkt und dient beispielsweise als Zwischenprodukt zur Herstellung von Pyrrolidonen.

Das nachfolgende Beispiel soll die Erfindung zusätzlich erläutern.

### Beispiele

### Beispiel 1

Die Prozentangaben sind durch Gaschromatographie ermittelte Gewichtsprozente (Methode mit innerem Standard).

### Herstellung einer 1,4-Butandiol enthaltenden Reaktionsmischung (Zulauf)

Der Zulauf wurde durch Hydrierung einer 30 %igen wässrigen Maleinsäure-Lösung an einem Katalysator gemäß Beispiel 1 der EP-A 848 991, der Platin, Silber, Rhenium und Eisen auf Aktivkohle enthielt, in der Flüssigphase bei 200 bar und Temperaturen von 180 bis 190°C gewonnen. Er hatte die folgende Zusammensetzung (Gew.-%): 2,5 % n-Butanol, 97,0 % 1,4-Butandiol, 0,2 % n-Propanol, 0,2 % 2-Hydroxytetrahydrofuran sowie Wasser und mehrere weitere Verbindungen, deren Gehalte unter 0,1 % lagen.

### Cyclisierung zu GBL

In einem Rohrreaktor wurden 100 ml ( 100 g) eines Katalysators bestehend aus 11 % CuO, 7 % CaO und 82 % SiO₂ gefüllt. Nach Aktivierung des Katalysators im Wasserstoffstrom wurde mittels einer außenliegenden Heizung so aufgeheizt, daß die Reaktorinnentemperatur 280°C betrug. Danach wurde bei einem kontinuierlichen Wasserstoffstrom (20 NL/h) der Zulauf (Zusammensetzung siehe oben) bei 1013 mbar über den Sumpf des Reaktors zugefahren (20 g/h). Nach 5 h stellte sich ein stationärer Zustand ein. Im gasförmigen Reaktionsaustrag fanden sich 2,0 % n-Butanol, 97,2 % Gamma-Butyrolacton, und 0,1 % n-Propanol, sowie einige weitere Produkte, deren Gehalt unter 0,1 % lagen und Wasser.

Die GBL-Ausbeute lag über 99 %. Der Katalysator zeigte auch nach mehr als 200 h keine Desaktivierungserscheinungen.

Ein Teil des Reaktionsaustrages wurde in einer 1 m Drehbandkolonne aufdestilliert (20 mbar). Die erzielte GBL-Reinheit lag dabei bei 99,9 %.

## Patentansprüche

1. Verfahren zur Herstellung von gamma-Butyrolacton (GBL) durch Umsetzung von 1,4-Butandiol an einem Kupferkatalysator, **dadurch gekennzeichnet, daß** eine 1,4-Butandiol enthaltende Reaktionsmischung, die weitere von 1,4-Butandiol verschiedene Alkohole und Wasser enthält, eingesetzt wird.

2. Verfahren nach Anspruch 1, **dadurch gekennzeichnet, daß** die Reaktionsmischung ein Hydrieraustrag der Hydrierung einer Verbindung ausgewählt aus 1,4-Butindiol, 4-Hydroxybutyraldehyd, 2,3- oder 2,5-Dihydrofuran, Maleinsäure, Maleinsäuremonoestern, Maleinsäurediestern, Maleinsäureanhydrid, und/oder einem der bei der Hydrierung entstehenden Zwischenprodukt ist.

3. Verfahren nach Anspruch 1 oder 2, **dadurch gekennzeichnet, daß** als Alkohol ein einwertiger, aliphatischer C₁-C₄-Alkohol eingesetzt wird.

4. Verfahren nach Anspruch 3, **dadurch gekennzeichnet, daß** die Reaktionsmischung als Alkohol, Methanol, Ethanol, Propanol oder n-Butanol enthält.

5. Verfahren nach einem der Ansprüche 1 bis 4, **dadurch gekennzeichnet, daß** die Umsetzung bei einer Temperatur von 200 bis 350°C durchgeführt wird.

6. Verfahren nach einem der Ansprüche 1 bis 5, **dadurch gekennzeichnet, daß** die Umsetzung in einem Druckbereich von 0,5 bis 10 bar durchgeführt wird.

7. Verfahren nach einem der Ansprüche 1 bis 6, **dadurch gekennzeichnet, daß** die Katalysatoren Kupfer allein,oder zusammen mit einem Metall der VIII. Nebengruppe des Periodensystems der Elemente auf einem Träger enthalten.

8. Verfahren nach Anspruch 7, **dadurch gekennzeichnet, daß** die Katalysatoren als Trägermaterial mindestens eine Verbindung ausgewählt aus den Oxiden des Aluminiums und Titans, Siliciumoxid, Zirkoniumoxid, Calciumoxid, Bariumoxid und Chromtrioxid enthalten.

9. Verfahren nach den Ansprüchen 1 bis 8, **dadurch gekennzeichnet, daß** die Umsetzung an fest angeordneten Katalysatoren in der Gasphase durchgeführt wird.

## Claims

1. A process for preparing gamma-butyrolactone (GBL) by reaction of 1,4-butanediol over a copper catalyst, in which a 1,4-butanediol-containing reaction mixture comprising further alcohols other than 1,4-butanediol and water is used.

2. A process as claimed in claim 1, wherein the reaction mixture is a hydrogenation product from the hydrogenation of a compound selected from the group consisting of 1,4-butynediol, 4-hydroxybutyraldehyde, 2,3- and 2,5 dihydrofuran, maleic acid, maleic monoesters, maleic diesters and maleic anhydride and/or an intermediate formed in the hydrogenation.

3. A process as claimed in claim 1 or 2, wherein a monohydric, aliphatic C₁-C₄-alcohol is used as alcohol.

4. A process as claimed in claim 3, wherein the reaction mixture comprises methanol, ethanol, propanol or n-butanol as alcohol.

5. A process as claimed in any of claims 1 to 4, wherein the reaction is carried out at from 200 to 350°C.

6. A process as claimed in any of claims 1 to 5, wherein the reaction is carried out in a pressure range of from 0.5 to 10 bar.

7. A process as claimed in any of claims 1 to 6, wherein the catalysts comprise copper alone or copper together with a metal of transition group VIII of the Periodic Table of the Elements on a support.

8. A process as claimed in claim 7, wherein the catalysts comprise, as support material, at least one compound selected from the group consisting of the oxides of aluminum and titanium, silicon oxide, zirconium oxide, calcium oxide, barium oxide and chromium trioxide.

9. A process as claimed in any of claims 1 to 8, wherein the reaction is carried out in the gas phase over fixed-bed catalysts.

## Revendications

1. Procédé de production de gamma-butyrolactone (GBL) par réaction du 1,4-butanediol sur un catalyseur au cuivre, **caractérisé en ce que** l'on met en oeuvre un mélange réactionnel contenant le 1,4-buranediol, qui contient d'autres alcools différents du 1,4-butanediol et de l'eau.

2. Procédé selon la revendication 1, **caractérisé en ce que** le mélange réactionnel est une décharge d'hydrogénation de l'hydrogénation d'un composé choisi parmi le 1,4-butynediol, du 4-hydroxybutyraldéhyde, du 2,3- ou du 2,5-dihydrofuranne, de l'acide maléique, de monoesters de l'acide maléique, de diesters de l'acide maléique, de l'anhydride maléique et/ou d'un des produits intermédiaires formés lors de l'hydrogénation.

3. Procédé selon la revendication 1 ou 2, **caractérisé en ce que** l'on met en oeuvre comme alcool, un alcool aliphatique monovalent en C₁-C₄.

4. Procédé selon la revendication 3, **caractérisé en ce que** le mélange réactionnel contient comme alcool, le méthanol, l'éthanol, le propanol ou le n-butanol.

5. Procédé selon l'une quelconque des revendications 1 à 4, **caractérisé en ce que** la réaction est réalisée à une température allant de 200 à 350°C.

6. Procédé selon l'une quelconque des revendications 1 à 5, **caractérisé en ce que** la réaction est réalisée dans un intervalle de pression allant de 0,5 à 10 bar.

7. Procédé selon l'une quelconque des revendications 1 à 6, **caractérisé en ce que** les catalyseurs contiennent le cuivre seul ou avec un métal du groupe secondaire VIII du système périodique des éléments, sur un support.

8. Procédé selon la revendication 7, **caractérisé en ce que** les catalyseurs contiennent comme support, au moins un composé choisi parmi les oxydes de l'aluminium et du titane, l'oxyde de silicium, l'oxyde de zirconium, l'oxyde de calcium, l'oxyde de baryum et le trioxyde de chrome.

9. Procédé selon l'une quelconque des revendications 1 à 8, **caractérisé en ce que** la réaction est réalisée en phase gazeuse sur le catalyseur solide.
